# EUROPEAN PATENT APPLICATION

(11) **EP 4 817 677 A1**
(43) Date of publication of application: **07.10.2026**
(21) Application number: 25168013.8
(22) Date of filing: 02.04.2025
(51) Int. Cl.: A61N 5/10

(54) **RADIATION THERAPY SYSTEM**

(71) Applicant: RaySearch Laboratories AB, 104 30 Stockholm (SE)
(72) Inventor: TRANEUS, Erik, 752 39 UPPSALA (SE); NILSSON, Rasmus, 115 44 STOCKHOLM (SE)

(57) **Abstract**

Disclosed herein is a method for providing a radiation therapy plan using pencil beam scanning for treatment of a target volume (18) in a patient, the method comprising the steps of:
- Providing a plurality of pencil beams (15, 15", 15‴, 15ʺʺ) from a pencil beam scanning radiation source (4), each pencil beam defining a spot (20", 20"', 20ʺʺ), each of the pencil beams (15, 15", 15‴, 15ʺʺ) having a set of particles with a first incident energy (N₁) for delivering a dose within the target volume (18); and
- Selecting spot energies and/or spot weights to be delivered using the first incident energy (N₁) in order to conform the plurality of spots (20", 20"', 20ʺʺ) from the pencil beam (15, 15", 15‴, 15"") to the target volume (18);
The method further comprises the steps of:
- Manipulating the spot energy of at least some of the plurality pencil beams by placing a movable object (22, 22', 22", 22‴, 22ʺʺ), at least partially, into the at least some of the plurality of pencil beams (15, 15", 15‴, 15ʺʺ) between the pencil beam scanning radiation source (4) and the patient; and
- Changing a position of the movable object (22, 22', 22", 22‴, 22"") from a first position (26a, 26a', 26a") to a second position (26a) during a treatment while maintaining the first incident energy (N₁), whereby a change from the first position (26a, 26a', 26a") to the second position (26a) leads to a change of the spot energy depth as seen in a direction along a central beam axis (c) of the plurality of pencil beams (15, 15", 15‴, 15ʺʺ).

## Description

### Technical field

This disclosure relates in a first aspect to radiation treatment and to optimization of radiation treatment plans using specialized objects that offer the possibility to modify a radiation beam, for example a pencil beam. In a second aspect this disclosure relates to a device that is designed to be arranged in a treatment room in between a pencil beam scanning radiation source and the patient, whereby the device comprises a three-dimensional object that can be moved in relation to a radiation beam to modify the energy of the radiation beam.

### Technical background

Proton Pencil Beam Scanning (PBS) is a method for delivering radiation therapy in which a narrow proton beam is scanned across a target volume. PBS irradiation further involves delivering radiation to a target volume by sequentially depositing a series of incident energies. This approach allows for precise deposition of radiation in three dimensions, comparable to "3D printing" the dose within the target region. Each incident energy corresponds to a particular proton energy spectrum that reaches a specific water-equivalent depth within the patient. Within each layer, multiple "spots" of radiation are delivered laterally across the target volume in a rapid scanning process. By stacking and layering these spot irradiations across various proton energies, a three-dimensional dose distribution can effectively be "printed" in the target, achieving high conformality and dose sparing of healthy tissue.

A characteristic feature of proton PBS delivery is the discrete stepping from one incident energy to the next. Although the spot delivery within a single incident energy is fast, the transition to a different proton energy often requires a non-negligible delay. These layer transitions can typically last from approximately 0.2 seconds up to 30 seconds, depending on the proton therapy machine and configuration. This is in part due to needed adjustments, especially in the magnets that guide and focus the beam from the accelerator to the treatment room. Given that a typical treatment beam may include up to 30 nominal energies, total delivery times for a single beam can extend to several minutes or more. This relatively prolonged time window can be problematic for tumors located in organs that move during the delivery, such as in the lung or near the heart, because organ motion during treatment can degrade dose conformity and reduce the target coverage of the intended treatment plan.

Various approaches have been explored to mitigate the negative effects of organ motion during proton PBS delivery. One possibility is to shorten the total delivery time dramatically (as in so-called "FLASH" irradiation) by employing a single incident energy of high-intensity beam current in conjunction with a beam-specific, patient-tailored three-dimensional object. Such a three-dimensional object is statically placed in the radiation beam path, between the radiation source and the patient, to shape or modulate the beam. Although this approach enables rapid dose delivery, the design and manufacturing process for such a patient-specific three-dimensional object can be expensive and time-consuming, limiting the practicality of this method in certain clinical scenarios.

Other motion-mitigation methods include breath-hold or gated delivery techniques, in which the beam is turned on only during short periods when the patient's anatomy is in a consistent, reproducible position. To enable longer breath-hold periods, some protocols call for oxygen ventilation to deplete carbon dioxide (CO₂) in the blood, thereby increasing the duration a patient can comfortably hold their breath. In addition, techniques have been developed to stimulate or synchronize beam delivery with the patient's natural respiratory cycle. These gating and synchronization strategies can reduce interplay effects between tumor motion and the scanned proton beam.

A further approach is the use of robust treatment planning based on four-dimensional computed tomography (4DCT) data. In this case, treatment plan optimization considers the range of possible anatomical variations over the respiratory cycle, thus reducing sensitivity to motion. However, these solutions often come with trade-offs in terms of plan complexity, treatment time, patient compliance, and cost. As proton therapy gains wider adoption for treating thoracic and abdominal tumors, addressing motion remains a significant challenge to ensure both treatment quality and clinical efficiency.

The use of a static, three-dimensional object tailor made to each patient, is disclosed for example in EP 3 957 361 A1.

Despite these measures, the need remains for improved solutions that address the challenges posed by organ motion during proton PBS therapy, without incurring excessive cost or complexity. In particular, the slow transition between nominal energies continues to limit the effectiveness of motion mitigation strategies, underscoring the importance of further technological improvements.

### Summary

Considering the above it is an object of this disclosure to provide a method for optimization of a radiation therapy treatment plan using pencil beam scanning, that is robust and flexible and can be used in many clinical scenarios.

It is further an object of this disclosure to provide a radiation therapy planning system designed to provide a robust and flexible radiation therapy treatment plan, which can be used in many clinical scenarios.

**In** here a method for providing a radiation therapy plan using pencil beam scanning for treatment of a target volume in a patient, is disclosed. The method comprises several steps. Initially, a plurality of pencil beams is provided from a pencil beam scanning radiation source. Each pencil beam defines a spot, and each of the pencil beams has a set of particles with a first incident energy for delivering a dose within the target volume. This approach allows for precise targeting of the radiation to the desired area within the patient. The incident energy is further explained below, it is basically the energy which the particle beam has when reaching the treatment room.

The method further involves selecting spot energies and/or weights to be delivered using the first incident energy in order to conform the plurality of spots from the pencil beam to the target volume. This selection step ensures that the radiation dose is tailored specifically to the shape and size of the target volume, maximizing treatment efficacy while minimizing damage to surrounding healthy tissue.

In another step a movable object is placed, at least partially, into at least some of the plurality of pencil beams between the pencil beam scanning radiation source and the patient in order to manipulate the spot energy of at least some of the pencil beams. The movable object is thereby placed between the pencil beam scanning radiation source and the patient. Using a movable object allows for a single incident energy to be used to cover the entire target volume and thereby avoiding that the incident energy at the incident beam needs to be changed, which invokes time consuming steps that including adjusting magnets along the path of the particles from the accelerator to the treatment room.

An aspect of the method involves changing a position of the movable object from a first position to a second position during a treatment while maintaining the first incident energy. A change from the first position to the second positions leads to a change of the spot energy depth as seen in a direction along a central beam axis of the plurality of pencil beams. Any subsequent discrete position, different from the first position and the second position, may of course be chosen, depending on the needs and requirements of the plan at hand. Thus, in other words, a sequence of positions may be provided for the plan, whereby the positions describe a specific location of the movable object over time or in a certain time-period.

It is to be noticed that the steps of the method do not need to be in the above-described sequence, in particular the positioning of the movable obj ect may be performed before selecting spot energies and/or spot weights or it may be done simultaneously to the selection of the spot energies and/or spot weights.

The movable object may be designed to extend into the plurality of pencil beams in the first- and/or the second position. Alternatively, it may be designed to only extend into some of the plurality of pencil beams in the first- and/or second position.

This dynamic adjustment capability allows for more precise control over the radiation dose distribution throughout the treatment process and it basically allows to change the delivered energy at the target volume by simply moving the movable object.

The method may further comprise directing the plurality of pencil beams perpendicular to a central beam axis so that the plurality of pencil beams and spots, respectively, cover the target volume in lateral directions. This ensures comprehensive coverage of the target volume from multiple angles.

This improves conformation of the plurality of pencil beams to the target volume. Changing the lateral directions, thus the directions oriented perpendicular to the central beam axis can be done very quickly, for example using magnets at the pencil beam scanning radiation source.

The step of changing positions of the movable object may be continued during the treatment depending on the geometry of the target volume, until the entire target volume is treated with spots using the first incident energy. If the entire target volume cannot be treated with spots using the first incident energy, a second incident energy may be added, and the previous steps may be repeated for the second incident energy.

Again, an infinite or plurality of discrete positions of the movable object may be chosen for the treatment, no matter if the first incident energy or the second incident energy is chosen.

This process allows for complete treatment of the target volume, even in cases where a single incident energy is insufficient.

The movable object may be moved translatory from the first position to the second position, along a first direction oriented at an angle to a central beam axis defined by at least one of the plurality of pencil beams. This translational movement provides an additional degree of freedom in modifying the spot energies. This translational movement allows for continuous adjustment of the spot energies throughout the treatment process.

Translatory in the above sense means that the movement happens along a line in a back-and-forth fashion. In an embodiment the line may be a straight line.

Alternatively, the movable object may be rotated around a central axis from the first position to the second position, wherein the central axis is parallel with a central beam axis defined by at least one of the plurality of pencil beams. This rotational movement allows for continuous adjustment of the spot energies throughout the treatment process.

The movable object may be designed as a wedge or a double wedge. This shape allows for gradual modification of the spot energies and positions as the wedge or double wedge is translatory moved or rotated.

In another embodiment the movable object may be designed as triple wedge, a quadruple wedge and so on.

It should be understood that a wedge in here means a thickness profile that can be either linear or any other shape, for example, following an exponential function. Thus, the wedge is not only a wedge in its traditional shape but it may also be a wedge that is formed having a curved surface.

The method may comprise more than one movable object in the form of wedges. Multiple movable objects, for example in the form of wedges, provide even greater flexibility in modifying spot energies across different regions of the target volume.

Further, using two wedges may be used to improve the spot positioning in the target volume as explained herein referring to the figures.

In some embodiments, the movable object may be designed as a disc defining a radial direction and the axial direction around which the disc is designed to rotate. The disc has a profiled part along its circumference in the axial direction, and this profiled part is designed to extend into at least some of the plurality of pencil beams. The axial direction is oriented parallel to a central beam axis defined by at least one of the plurality of pencil beams. This disc design allows for continuous and precise adjustment of spot energies as the disc rotates.

The disc may be a disc without a hole in the middle or with a hole in the middle.

The disclosure also covers a radiation therapy planning system for providing a radiation therapy plan for treatment of a target volume in a patient. This system comprises a pencil beam scanning radiation source for providing a plurality of pencil beams, each of the pencil beams defining a spot and having a set of particles with a first incident energy for delivering a dose distribution within the target volume.

The radiation therapy planning system includes a processor and a memory for selecting spot energies and/or spot weights to be delivered using the first incident energy in order to conform a plurality of spots from the plurality of pencil beams to the target volume.

A movable object is placed, at least partially, into at least some of the plurality of pencil beams between the pencil beam scanning radiation source and the patient. The object is designed to manipulate or modify a spot energy of at least one of the spots based on the calculated and optimized spot energies.

The radiation therapy planning system further comprises a mechanism for changing a position of the movable object from a first position to a second position during the treatment, while maintaining the first incident energy. The change from first position to the second position leads to a change of the spot energy depth as seen in direction along a central beam axis (c) of the at least some of the plurality of pencil beams. This means in other words that different delivered energies reach the target volume since the movable object may alter the particles energy when it is inserted into at least some of the plurality of pencil beams.

In some embodiments, the mechanism allows the movable object to move back and forth along a first direction arranged at an angle to a central beam axis defined by at least one of the pencil beams. This translational movement capability provides flexibility in adjusting spot energies across different regions of the target volume.

The first direction may be a line or a straight line and the movement may be translatory.

Alternatively, the mechanism may allow the movable object to rotate around an axial direction, with the axial direction being arranged parallel to a central beam axis defined by at least one of the pencil beams. This rotational capability allows for continuous adjustment of spot energies throughout the treatment process and can be beneficial in terms of speed when changing from the first position to the second position.

The movable object in the system may be designed as a wedge or double wedge. This shape allows for gradual modification of spot energies as the movable object is moved or rotated.

The system may comprise at least two movable objects in the shape of wedges or double wedges. Multiple movable objects provide even greater flexibility in modifying spot energies across different regions of the target volume, as they can modify the geometry of several spots even better.

In some embodiments of the radiation therapy planning system, the movable object may be designed as a disc defining a radial direction and the axial direction around which the disc is designed to rotate. The disc has a profiled part along its circumference in the axial direction, and this profiled part is designed to extend into at least some of the plurality of pencil beams. The axial direction is oriented parallel to a central beam axis defined by at least one of the plurality of pencil beams. This disc design allows for continuous and precise adjustment of spot energies as the disc rotates and it may increase speed of adjustment between the first and second position.

There may be further positions such as third-, fourth- etc. positions depending on the needs to cover the entire target volume.

The disclosure also provides a computer program product comprising computer readable means which, when executed in a computer, will cause the computer to perform the method described above. This software implementation allows for easy integration of the improved radiation therapy planning method into existing treatment systems.

The method and system described in this disclosure offer several potential benefits. By allowing for dynamic adjustment of spot energies during treatment, they may provide more precise control over the radiation dose distribution within the target volume. The ability to use a single incident energy for treating the entire target volume, when possible, may also lead to more efficient treatments, potentially reducing treatment times and improving patient comfort. Furthermore, the flexibility provided by different designs of movable objects (wedges, double wedges, discs) allows for customization of the system to meet specific treatment requirements or constraints without the need for three-dimensional printing custom made objects that are placed in between the pencil beam scanning radiation source and the patient.

In this disclosure, certain terms and technical specifications will be used. Some of them are explained referring to the figures and some of them are herewith explained and specified for optimal understanding of the described disclosure:

### Pencil Beam

A pencil beam in radiation therapy refers to a narrow, focused beam of radiation particles, typically protons or heavier ions such as oxygen (O) ions, carbon (C) ions, helium (He) ions, neon (Ne) ions and lithium (Li) ions to deliver a precise dose of radiation to a target volume. Each pencil beam defines a spot, which is a specific area within the target volume that receives radiation depending on water equivalent path length (WEPL) and Bragg peak. The pencil beam scanning technique allows for the modulation of the beam's intensity and energy, enabling the delivery of a conformal dose distribution that matches the shape of the target volume. The dose distribution is thereby more or less "3D-printed" over the shape of the target volume using the spots. The pencil beam can be directed from the pencil beam scanning radiation source using magnets so that different spots can be delivered. The spots delivered by a pencil beam can be varied in energy (energy of ions), position and weight (number of ions). In a broad sense, pencil beams are used to treat complex-shaped tumors with high precision, minimizing damage to surrounding healthy tissues. In a narrow sense, each pencil beam is characterized by its incident energy and direction, which determines the depth of penetration and dose deposition within the target volume. A pencil beam is, as the name says, formed as a pencil beam with a diameter of about 0.5 to 20 millimeters. The treatment where pencil beams are typically used is called pencil beam scanning (PBS). The pencil beam is a scanned pencil beam.

### Pencil Beam Scanning

Pencil beam scanning also known as active scanning ion beam therapy. The process involves delivering patient irradiation as a sequence of quasi-monoenergetic "spots" with selected energy, direction, and weight to adequately cover the treatment volume. Pencil Beam Scanning (PBS) is an advanced proton therapy technique that uses a narrow, magnetically steered proton beam to deliver radiation in finely controlled spots across a tumor or target volume in three dimensions. In this method, thousands of individual radiation spots are precisely overlapped-layer by layer and spot by spot-to "3D print" a tailored dose that conforms closely to the tumor's and target volume's shape, thereby minimizing exposure to surrounding healthy tissue. PBS can be defined as a method and system for achieving superior dose conformity in radiation therapy by dynamically steering a pencil-thin proton beam to selectively irradiate tumor tissues, eliminating the need for fixed hardware components and allowing for customizable energy modulation to adapt to varying tumor depths and complex anatomical structures.

### Movable Object

The movable object in the context of this disclosure is a three-dimensional object that can be positioned within the path of the pencil beams. Since the position of the movable object can be changed, delivered spot energies can be manipulated or modified.. In its widest interpretation the movable object may be a three dimensional object having a thickness as seen along a central beam axis. The movable object may be made of plastic, metal or any other suitable and homogenous or non-homogenous material. In a broad sense, the movable object can take various forms, such as a wedge, a pyramid, a prism, a double wedge, a disc with a profile, or other shapes, and can be moved translationally, for example along a linear line, or rotationally to achieve the desired modification of the energies delivered in the target volume and the incident energy. Discrete positions of the movable object during the treatment are used to optimize dose delivery over the target volume.

### Incident energy

Incident energy refers to the initial energy level of the particles in a pencil beam as they are emitted from the pencil beam scanning radiation source. This energy normally determines the depth of penetration and the dose distribution within the target volume. In here however the incident energy is modified using the movable object and thereby the actual delivered energies can be varied, even though the pencil beam scanning radiation source emits particles with a constant incident energy. In a broad sense, incident energy is a parameter in radiation therapy planning that influences the ability to conform the radiation dose to the shape and size of the target volume. In a narrow sense, the incident energy is maintained during treatment, while the movable object is used to modify the spot energies to achieve optimal dose delivery. The unit for incident energy is typically expressed in MeV (mega electron volts), MeV/n (mega electron volts per nucleon) or MeV/u (mega electron volts per unit mass or unit atomic mass). This disclosure however covers using more than one incident energy if the target volume or treatment plan requires so. Finally, an incident energy generally comprises ions and/or particles, which have a spectrum "around" the incident energy value, for example a gaussian distribution with mean 150 Mev and 4 MeV standard deviation width. It is therewith not an absolute term and particles or ions, as described herein, may follow for example a gaussian distribution around a specific incident energy value. Further, in here the incident energy is different from the one coming directly out from the accelerator. The energy coming out directly from the accelerator is guided with magnets and other elements to the pencil beam scanning radiation source (see below) and the energy exciting form the radiation source is the incident energy. These energies, thus the one coming directly from the accelerator and the incident energy may be similar but not necessarily the same.

### Spot

In pencil beam scanning (PBS) for proton or other ion therapy, a "spot" is defined as a pencil beam with defined energy and direction. Each spot is typically characterized by a beam diameter of about 0.5 to 20 mm, with energy deposited until the depth of the Bragg peak to target a precise depth, optimally within the target volume. These spots are methodically arranged in a grid or customized pattern across tumor layers or target volume layers, allowing for individual intensity modulation to tailor the dose distribution. This enables "dose painting," or "dose 3D printing", whereby hundreds or thousands of spots are superimposed to create a uniform, conformal radiation treatment that maximizes tumor (target volume) coverage while minimizing exposure to surrounding healthy tissue.

In ion beam therapy or PBS, a "spot" refers to a collection of ions of a specific energy level at a particular lateral location and direction. The number of particles delivered to a spot is referred to as the "spot weight" (c.f. below).

A spot may be defined as a singular, controlled radiation delivery unit in PBS, characterized by its specific size, weight, energy and spatial arrangement all of which contribute to its ability to conform radiation doses precisely to complex tumor geometries or target volumes.

### Spot Energy

Spot energy is the energy level or delivered energy of a specific spot within the target volume that is irradiated by a pencil beam. It is calculated and optimized to conform the dose distribution to the target volume. In a broad sense, spot energy and spot weight optimization is a key aspect of pencil beam scanning, allowing for precise control over the dose delivered to different areas of the target volume.

Each spot in ion therapy represents a collection of ions with a specific energy level and lateral position.

The spot energy determines the location of the Bragg peak, which is the position where the maximal energy deposition happens as the protons in the spot get stopped by the patient medium, preferably in the target volume. In this disclosure, the spot energy can be adjusted by the movable object, which modifies the energy of the pencil beams based on the calculated and optimized spot energies.

### Spot Weight

Spot weights are defined as the number of particles to be delivered at a specified energy level to that lateral position within the target volume. During treatment planning, spot weights are used as optimization variables to achieve the desired dose distribution. The optimization process involves distributing desired doses over predefined spots, assigning each a weight according to the dose needed at that location. If a spot's weight falls below a particular minimum threshold, indicative of its insignificance or non-utility, it may be discarded, and its weight redistributed to more significant spots. This redistribution ensures dose optimization and adherence to constraints of the radiation therapy planning or delivery system.

### Target Volume

The target volume is the specific area within a patient's body that requires radiation treatment and could for example correspond to a tumor and other potential cancer cells. It is the focus of the radiation therapy plan and is defined by its shape, size, and location. The target volume encompasses the tumor and any surrounding tissues that may be at risk of containing cancerous cells. Finally, the target volume is the area that the pencil beams are conformed to, using optimized spot energies to deliver a precise and effective dose of radiation.

### Pencil beam scanning radiation source

The pencil beam scanning radiation source is the device or apparatus where the pencil beams used in the treatment exit towards the patient. It provides the initial incident energy for the scanned pencil beams. The pencil beam scanning radiation source is positioned so that is it has a direct line of sight towards the patient and the movable object, without major obstacles along the way.

Exemplary, the pencil beam scanning radiation source may be positioned in the same room as the patient and the movable object. Alternatively, the pencil beam scanning radiation source may be arranged in one room and the patient and the moveable object in another room. Regarding the pencil beam scanning radiation source, the sequence how ions or protons move may be accelerator - magnets and other guiding elements - pencil beam scanning radiation source - movable object - patient/target volume. Finally, the movable object is not necessarily a part of the pencil beam radiation source, typically it is not a part of the pencil beam radiation source. The pencil beam scanning radiation source may be called radiation source.

### Energy layer /Delivered energies

The term energy layer or delivered energies in here refers to the actual delivered energy of the ions, which result in the spots in the target volume. A delivered energy comprises of several spot energies based on a specific control point. The spots and spot energies, respectively, are typically arranged in energy layers within the target volume. These energy layers may be called "delivered energies". The incident energy differs from energy layers or delivered energies in that the movable object has absorbed a "delta" of the incident energy at the exit of the pencil beam scanning radiation source, which results in a lower "energy layer" or "delivered energy" within the target volume. The energy layer or delivered energy is therewith the energy that is actually and finally delivered with the target volume within the patient.

### Brief Description of the Drawings

The disclosure will be described in more detail in the following, by way of example and with reference to the appended drawings, in which
- Fig. 1:: schematically illustrates a setup for radiation treatment;
- Fig. 2a:: schematically illustrates a pencil beam scanning treatment according to the prior art;
- Fig. 2b:: schematically illustrates a pencil beam scanning treatment similar to figure 2a also according to the prior art;
- Fig. 3a: schematically illustrates a radiation therapy planning system according to one embodiment of this disclosure;
- Fig. 3b: schematically illustrates a similar set up as figure 3a also according to this disclosure;
- Fig. 4a: schematically illustrates a radiation therapy planning system according to another embodiment of this disclosure;
- Fig. 5: schematically illustrates an example of a movable object;
- Fig. 6: schematically illustrates another example of a movable object;
- Fig. 7: schematically illustrates a radiation therapy planning system according to another embodiment of this disclosure;
- Fig. 8: schematically illustrates a radiation therapy planning system according to still another embodiment of this disclosure;
- Fig. 9: schematically illustrates some components of the radiation therapy planning system according to this disclosure; and
- Fig. 10: schematically illustrates a method according to this disclosure.

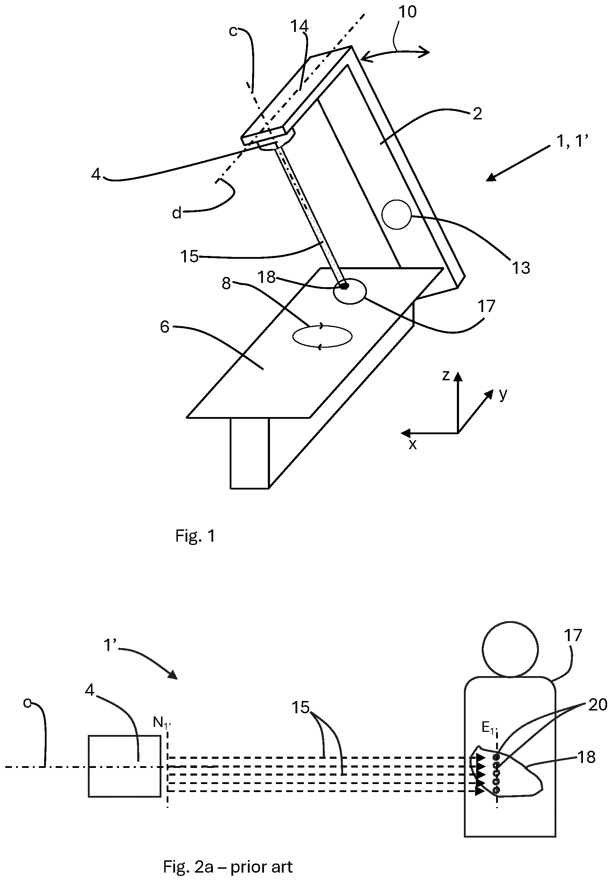

Turning now to the figures, which show different aspects and concepts of the disclosure, figure 1 schematically illustrates a radiation therapy treatment system 1 comprising a gantry 2 having a pencil beam scanning radiation source 4. The pencil beam scanning radiation source 4 is basically where beams 15 leave the pencil beam scanning radiation source 4 in the patient room 1' and the pencil beam scanning radiation source 4 is supplied with ions from an accelerator (not shown in figure 1). The radiation therapy treatment system 1 may a be used for stereotactic radio surgery (SRS) using pencil beams. The radiation therapy treatment system 1 may further comprises a patient couch or couch 6, which is used to place a patient when performing radiation treatment on such a patient. For illustrative purposes the radiation therapy treatment system 1 is shown without a patient. The couch 6 can be rotated around an axis in order to place the patient optimally during or for the treatment. In the example illustrated in figure 1, the couch angle 8 can be adjusted by rotating the couch 6 around the z-axis. Other movements of the couch 6 may be possible. A central beam axis c depends on the position and therewith a gantry angle 10, since the gantry 2 can rotate around a centre of rotation 13. When the pencil beam 15 is allowed to extend from the pencil beam scanning radiation source 4 towards a body part 17 of a patient, said body part 17 comprising a target or target volume 18, then the radiation beam 15 travels along the central beam axis c, while its shape in a cross-sectional plane is perpendicular to the central beam axis c, and depends on the diameter of the pencil beam 15. The gantry may be capable of rotating all 360° around the centre of rotation 13 for irradiating of a target (not shown in figure 1) from different gantry angles 10. The gantry 2 may rotate around a y-axis as shown in figure 1. For better reaching targets the pencil beam scanning radiation source 4 may be mounted on an extension arm 14 or the like, which extension arm 14 is part of the gantry 2.

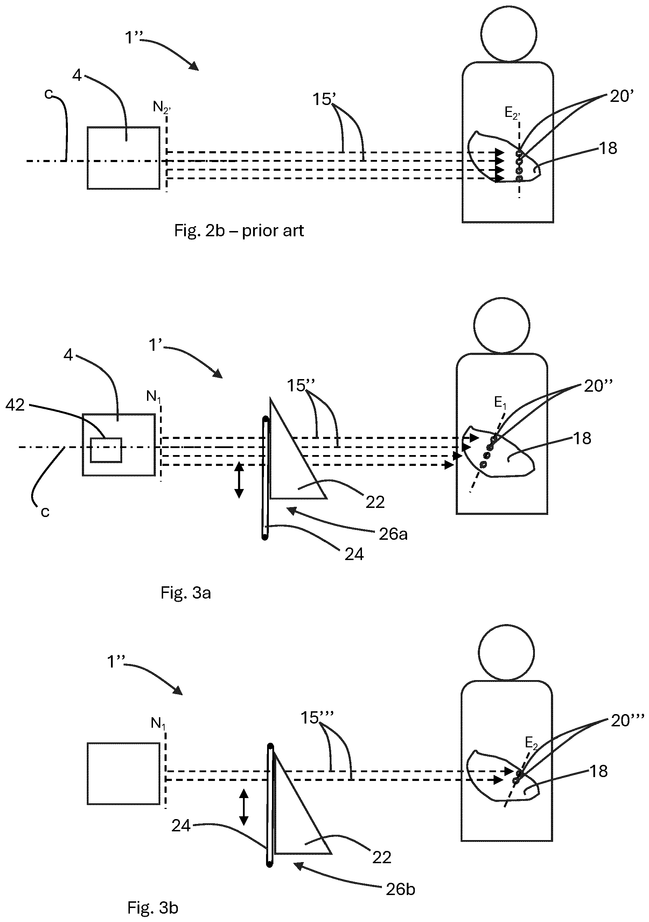

Figures 2a and 2b illustrate a pencil beam scanning treatment according to the prior art and how it is typically done today, if no customized three-dimensional object is placed in the pencil beams 15. It is to be noted that even though figures 2a and 2b illustrate side view onto the radiation treatment system 1' or radiation treatment planning system 1', it is clear the pencil beams 15 illustrated in the figures area also distributed perpendicular to surface determined by the figures 2a and 2b. The same is true for any subsequent figures.

Figure 2a schematically illustrates how a target volume 18 in a patient or body part 17 is treated using a plurality of pencil beams 15 emitted along the central beam axis c. The pencil beams 15 and ions, respectively, are emitted from the pencil beam scanning 4 at a first incident energy N_{1'} and travel a distance towards the target volume 18 where they are then deposited in discrete spots 20 at a first energy E_{1'} and at a specific depth within the target volume. The delivered energy E₁ comprises of several spot energies of each of the spots 20. Magnets or the like may be used to shift the lateral positions of the spots 20, whereby such lateral positions are characterized by being oriented perpendicular to the central beam axis c. The first incident energy N_{1'} and the first delivered energy E_{1'} are indicated in figure 2a. Now as these spots 18 are painted or 3D printed in the target volume 18, the first delivered energy E_{1'} needs to be modified in order to reach various depths within the target volume 18.

Such a change in delivered energy or energy layer is illustrated in figure 2b, which shows a similar illustration as figure 2a but this time with slightly changed pencil beams 15' comprising ions or particles with a different second incident energy N_{2'}. The central beam axis c is also indicated in figure 2b. The reason why a second incident energy N₂ is needed is because the delivered energy needs to be "deeper" (farther away from the pencil beam scanning radiation source 4) in the target volume 18 and this can only be achieved by changing the first incident energy N_{1 '}to a second incident energy N_{2'}, which is higher than the first incident energy N₁ so that the ions and pencil beams 15' can deliver ions at a second delivered energy E_{2'}.

It is to be noted that herein the term delivered energy is used, this term may however be replaced by energy layer or delivered energy layer. The delivered energy or energy layer or delivered energy layer each comprise several spot energies of each of the spots.

Now the issue with changing the incident energy from the first incident energy N_{1'} to the second incident energy N_{2'} is that this is time consuming as the incident energy is changed at the accelerator and since guiding elements such as guiding magnets and the like need to be adjusted along the way from the accelerator to the pencil beam scanning radiation source 4. This can be avoided by the embodiments illustrated in this disclosure.

Figure 3a illustrates a similar situation as figure 2a but in this embodiment a movable object 22 in the form of a wedge is placed in the pencil beams 15", which effects in the first delivered energy E₁ based on the first incident energy N₁. The distribution of the spots 20" is now however inclined due to the shape of the wedge 22/movable object. Since the movable object is reducing the energy of the particles or ions in the pencil beams 15, because they have to travel through the material of the wedge 22, the first delivered energy E₁ is basically dependent on the position of the wedge 22 and the first incident energy N₁. In figure 3a the movable object in the form of the wedge is shown in the first position 26a. It is to be noted that in this example the first energies of figure 2a and 3a do not necessarily need to have the same value.

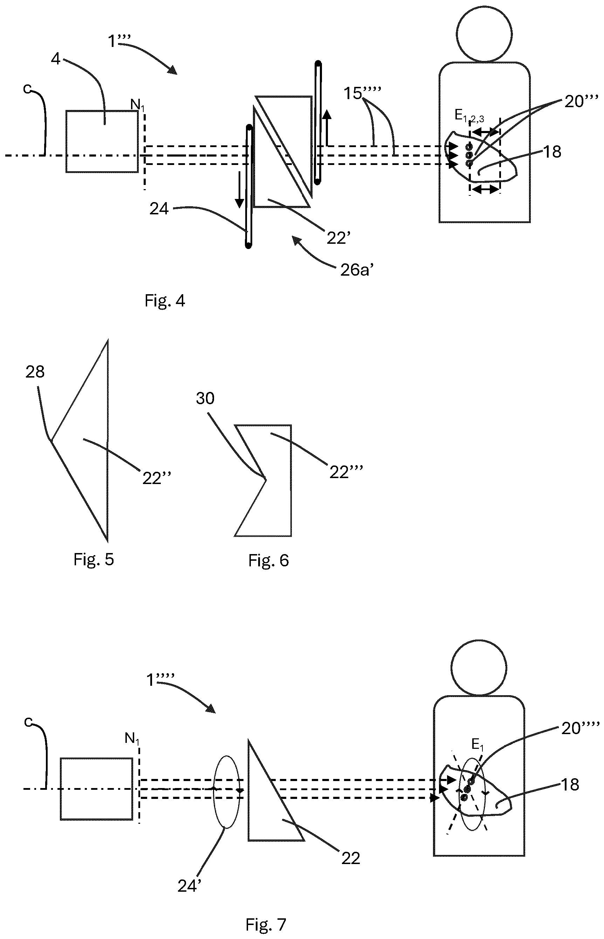

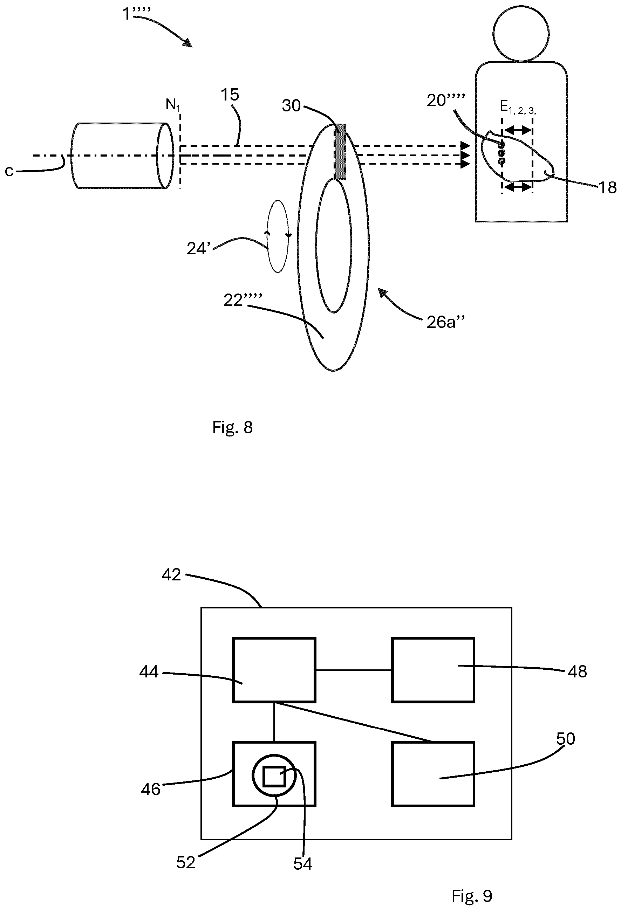

The movable object 22 is mounted on a mechanism 24 that is designed to move the movable object 22 in a translatory fashion. The mechanism 24 may be a conveyer belt like mechanism, a magnetic mechanism, a telescopic mechanism or any other suitable mechanism to translatory move an object. The double arrows in figure 3a illustrate how the wedge 22 can move back and further along a straight line. It is to be noted that any other shape of the movable object may be mounted and used on the mechanism 24, as indicated referring to figures 5 and 6. Finally figure 3a also discloses electronic components 42 of the radiation therapy system 1, 1", which electronic components 42 are going to be further explained referring to figure 9. Any of the embodiment illustrated herein may comprise the electronic components 42.

Figure 3b illustrates how the movable object 22 has been moved to a second position 26b, in which second position 26b less material of the wedge 22 is placed in the pencil beams 15‴, which results in the delivery of the spots 20‴ with a second delivered energy E₂ while keeping the first incident energy N₁. The spots 20‴ are also delivered inclined due to the shape of the movable object 22 in the form of a wedge 22. From figures 3a and 3b it becomes clear that the placement of the movable object 22 immediately improves and accelerates the treatment since no incident energy needs to be changed or at least much less nominal energies are needed to treat the entire target volume 18. Another advantage is that the movable object 22 is not custom made for each patient and can be used for different patients and treatments, which increases flexibility and reduces cost.

Various solutions will now be discussed referring to figures 4 to 8, which all use a similar inventive thought but illustrate different embodiments. Again, the skilled person will understand that various shapes of the movable object and the mechanism to move the movable object may be chosen.

Turning now to figure 4, another embodiment of the radiation therapy system 1‴ is shown, this time with more than one movable object 22', each of them having their own mechanism 24 for displacement along the double arrow. Using two or more movable objects 22', in the example embodied as wedges, has the advantage that the spots 20‴ and the delivered energies E_{1,2,3} etc, respectively are distributed perpendicularly to the central beam axis c and not inclined as shown in figures 3a and 3b. By moving the wedges out of their first positions 26a', thus towards or away from one another, the delivered energies E_{1, 2, 3} and the spots 20‴ can be easily moved to cover the entire target volume 18as indicated with the double arrows. **In** figure 4, the double arrows at the target volume 18 actually indicate how the spots 20‴ are moved away from the pencil beam scanning radiation source 4, when the wedges 22' move their thicker parts out of the pencil beams 15ʺʺ as indicated by the arrows next to the wedges 22'. Opposite movements of the wedges 22' is also allowed, thereby the spots 20‴ are moved closer to the pencil beam scanning radiation source 4 in the target volume.

Other shapes of movable objects 22", 22‴ in the form of a double wedge 22" with a middle ridge 28, as shown in figure 5 and a double wedge 22‴ with a middle valley 30, as shown in figure 6, may also be employed. These double wedges 22", 22‴ may be moved along straight translatory line, as described referring to figures 3a to 4, or they may be rotated as shown in figures 7 to 8. The advantage of using double wedges 22", 22‴ according to figures 5 and 6 is that the distance that the double wedges 22", 22‴ need to travel from a first to a second position, is reduced, which potentially makes the transition to different delivered energies even faster.

In figure 7, the movable object 22, is the wedge 22 as illustrated in figures 3a and 3b, however in this embodiment the mechanism 24' is designed to rotate the wedge 22 around the central beam axis c. The spot 20ʺʺ distribution with the delivered energy E₁ therewith also rotates in the target volume 18. This set up of the radiation therapy system 1ʺʺ can of course be combined with any of the ones illustrated in figures 3a to 4, in order to move the rotation in and out of the target volume 18, depending on the needed configuration. Any other shape of the movable object 22 may be designed to be mounted on a mechanism 24' that rotates it.

In addition, any of the solutions illustrated in the figures 3a to 8 may be combined with range-shifter slabs. Range shifter slabs have basically the same effect as the embodiment illustrated in figure 4, but range shifter slabs are basically plates that have the same thickness over their extension in a direction parallel to the central beam axis c.

Figure 8 illustrates a similar embodiment as figure 7, however the movable object 22ʺʺ is designed as a wheel 22ʺʺ that rotates around and axis which is parallel but offset to the to the central beam axis c. The wheel axis can also be tilted relative to the central beam axis. In the embodiment of figure 8, the wheel 22ʺʺ has a profile 30 that extends in the direction of the central beam axis c. In the embodiment in figure 8, this profile may be a circumferentially increasing thickness 30. This affects the delivered energies E_{1,2,3} as illustrated, by moving the spots 20ʺʺ in and out of the target volume 18, depending on the thickness of the profile 30 and therewith also the first position 26a" and any potential second, third or subsequent positions. The wheel 22ʺʺ therewith affects the pencil beams 15 with the profile 30. It is to be noted that figure 8 is partially shown in perspective to better illustrate the embodiment of the movable object 22ʺʺ. Other shapes of the wheel, for example with a staircase like profile (not shown), a part that is flat in the profile and any combinations thereof may be feasible and be used.

Finally, while the embodiments of figures 3a to 8 are shown as physical embodiments, these embodiments may actually be employed in a simulation, for example virtually, and therewith in a radiation therapy planning system and not only in a radiation therapy system.

The mechanisms 24 disclosed herein are designed to move, for example linearly move, the movable object in a first direction, which can be oriented perpendicular in regard to the central beam axis c. However, the first direction may also be oriented at an angle to the central beam axis c.

Figure 9 illustrates further electronic components 42 of the radiation therapy system or radiation therapy planning system, the electronic components 42 comprising a processor 44, a memory 46 connected to the processor 44, an optional extra memory 50 also connected to the processor 44 and/or the memory 46 and an input/output interface 48 having a display and/or a keyboard for user interaction. The processor 44 may be any combination of a central processing unit (CPU), multiprocessor, digital signal processor (DSP), an ASIC (application specific integrated circuit) capable of executing software instructions stored on the memory and capable of executing the methods described according to the embodiments herein. The memory 46 and/or the extra memory 50 may comprise software code in the form of a computer program product 52 comprising computer software 54 or instructions that comprise any method according to this disclosure. The computer software 54 may be executed by the processor 44.

The memory 46 and the extra memory 50 may be any of a random access memory (RAM) or read only memory (ROM) or any combination thereof.

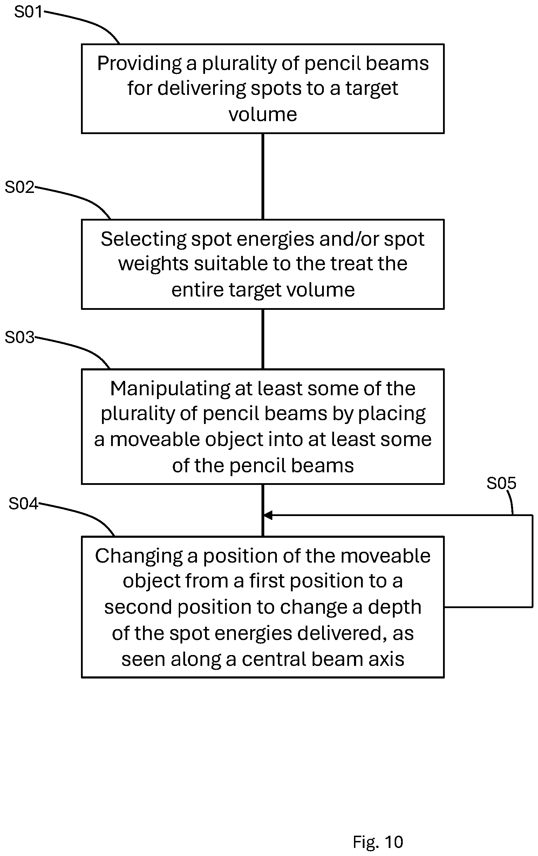

Figure 10 illustrates a method according to the disclosure, said method comprising the steps of:
- Providing S01 a plurality of pencil beams from a pencil beam scanning radiation source, each pencil beam defining a spot, each of the pencil beams having a set of particles with a first incident energy for delivering a dose within the target volume, and
- Selecting S02 spot energies to be delivered using the first incident energy in order to conform the plurality of spots from the pencil beam to the target volume.
The method may further comprise the steps of:
- Manipulating S03 the spot energy of at least some of the plurality of pencil beams by placing a movable object, at least partially, into the at least some of the plurality of pencil beams between the radiation source and the patient; and
- Changing S04 a position of the movable object from a first position to a second position during a treatment while maintaining the first incident energy, whereby a change from the first position to the second position leads to a change of the spot energy depth as seen in a direction along a central beam axis (c) of the at least some of the plurality of pencil beams.

The method may comprise the additional step of continuing or repeating S05 the changing positions into several other discrete positions of the movable object during the treatment depending on a geometry of the target volume until the entire target volume is treated with spots using the first incident energy and wherein, if the entire target volume cannot be treated with spots using the first layer, a second incident energy is added, and the previous steps are repeated for the second incident energy.

The movement of the movable object as disclosed herein, may be translatory following a curved or straight line or it may be rotational, rotating around an axis that is at least parallel to the central beam axis c.

Referring back to the figures it is to be noted that even though the concept has been explained using a first and a second position of the movable object, it is clear that a treatment may comprise many more such positions to cover the entire target volume.

The disclosure has now been described referring to embodiments disclosed above and in the enclosed figures. However, and as the skilled person may understand other embodiments than the ones disclosed herein may be conceivable and are equally possible within the scope of the disclosure, as defined by the appended patent claims.

## Claims

1. A method for providing a radiation therapy plan using pencil beam scanning for treatment of a target volume (18) in a patient, the method comprising the steps of:
- Providing a plurality of pencil beams (15, 15", 15"', 15ʺʺ) from a pencil beam scanning radiation source (4), each pencil beam defining a spot (20", 20"', 20ʺʺ), each of the pencil beams (15, 15", 15"', 15ʺʺ) having a set of particles with a first incident energy (N₁) for delivering a dose within the target volume (18);
- Selecting spot energies and/or spot weights to be delivered using the first incident energy (N₁) in order to conform the plurality of spots (20", 20"', 20"") from the pencil beam (15, 15", 15‴, 15ʺʺ) to the target volume (18);
wherein the method further comprises the steps of:
- Manipulating the spot energy of at least some of the plurality pencil beams by placing a movable object (22, 22', 22", 22"', 22ʺʺ), at least partially, into the at least some of the plurality of pencil beams (15, 15", 15‴, 15ʺʺ) between the pencil beam scanning radiation source (4) and the patient; and
- Changing a position of the movable object (22, 22', 22", 22‴, 22"") from a first position (26a, 26a', 26a") to a second position (26b) during a treatment while maintaining the first incident energy (N₁), whereby a change from the first position (26a, 26a', 26a") to the second position (26a) leads to a change of the spot energy depth as seen in a direction along a central beam axis (c) of the plurality of pencil beams (15, 15", 15"', 15ʺʺ).

2. The method according to claim 1, wherein the step of providing a plurality of pencil beams (15, 15", 15‴, 15ʺʺ) further comprises directing of these pencil beams perpendicular to the central beam axis (c) so that the plurality of pencil beams (15, 15", 15"', 15"") and spots (20", 20‴, 20ʺʺ), respectively, cover the target volume (18) in lateral directions.

3. The method according to claim 1 or 2, wherein the step of changing positions of the movable object (22, 22', 22", 22‴, 22ʺʺ) is repeated during the treatment depending on a geometry of the target volume (18) until the entire target volume (18) is treated with spots (20", 20"', 20ʺʺ) using the first incident energy (N₁) and wherein, if the entire target volume (18) cannot be treated with spots using the first incident energy, a second incident energy (N₂) is added, and the previous steps are repeated for the second incident energy (N₂).

4. The method according to any of claims 1 to 3, wherein the movable object (22, 22', 22", 22‴) is moved translatory from the first position (26a, 26a') to the second position (26b) along a first direction oriented at an angle to a central beam axis (c).

5. The method according to any of claims 1 to 3, wherein the movable object (22, 22", 22"', 22"") is rotated around a central axis from the first position (26a') to the second position, and wherein the central axis is parallel with a central beam axis (c).

6. The method according to claim 4 or 5, wherein the movable object is designed as a wedge (22) or a double wedge (22", 22‴').

7. The method according to claim 4 or 5, further comprising more than one movable objects in the form of wedges (22').

8. The method according to claim 5, wherein the movable object is designed as a disc (22ʺʺ) defining a radial direction and the axial direction around which the disc is designed to rotate, said disc having a profiled part (30) along its circumference in the axial direction, said profiled part being designed to extend into at least some of the plurality of pencil beams (15) and wherein the axial direction is oriented parallel to a central beam axis (c).

9. A radiation therapy system for providing a radiation therapy plan for treatment of a target volume (18) in a patient comprising:
- a pencil beam scanning radiation source (4) for providing a plurality of pencil beams (15, 15", 15‴, 15ʺʺ), each of the pencil beams (15, 15", 15‴, 15ʺʺ) defining a spot (20", 20"', 20"") and having a set of particles with a first incident energy (N₁) for delivering a dose distribution within the target volume (18);
- a processor (44) and a memory (46) for selecting spot energies and/or spot weights to be delivered using the first incident energy (N₁) in order to conform a plurality of spots (20", 20‴, 20ʺʺ) from the plurality of pencil beams (15, 15", 15‴, 15ʺʺ) to the target volume (18),
further comprising:
- a movable object (22, 22', 22", 22"', 22ʺʺ) placed, at least partially, into at least some of the plurality of pencil beams (15, 15", 15‴, 15ʺʺ) between the pencil beam scanning radiation source (4) and the patient, the movable object (22, 22', 22", 22"', 22ʺʺ) being designed to manipulate the spot energy of at least some of the plurality pencil beams;; and
- a mechanism (24, 24') for changing a position of the movable object from a first position (26a, 26a', 26a") to a second position (26b) during the treatment, while maintaining the first incident energy (Ni), whereby a change from the first position (26a, 26a', 26a") to the second position (26b) leads to a change of the spot energy depth as seen in a direction along a central beam axis (c) of the at least some of the plurality of pencil beams (15, 15", 15"', 15ʺʺ).

10. The radiation therapy system according to claim 9, wherein the mechanism (24) is allowing the movable object (22, 22', 22", 22"', 22ʺʺ) to move back and forth along and stop at any position along a first direction arranged at an angle to a central beam axis (c) defined by at least one of the pencil beams (15, 15", 15"', 15ʺʺ).

11. The radiation therapy system according to claim 9, wherein the mechanism (24') is allowing the movable object (22, 22ʺʺ) to rotate around an axial direction, the axial direction being arranged parallel to the central beam axis (c).

12. The radiation therapy system according to claim 10 or 11, wherein the movable object is designed as a wedge (22) or double wedge (22", 22"').

13. The radiation therapy system according to claims 12, comprising at least two movable objects in the shape of wedges (22, 22') or double wedges (22", 22"').

14. The radiation therapy system according to claim 11, wherein the movable object is designed as a disc (22ʺʺ) defining a radial direction and the axial direction around which the disc is designed to rotate, said disc having a profiled part (30) along its circumference in the axial direction, said profiled part being designed to extend into at least some of the plurality of pencil beams (15) and wherein the axial direction is oriented parallel to the central beam axis (c).

15. The radiation therapy system according to any of the previous claims 9 to 14, further comprising a patient bed (6) and a gantry (2), said gantry (2) comprising the pencil beam scanning radiation source (4), the mechanism (24, 24') and the movable object (22, 22', 22", 22‴, 22ʺʺ).

16. The radiation therapy planning system according to any of the previous claim 9 to 15 wherein the radiation therapy system is a radiation therapy planning system, designed to simulate the use of the movable object (22, 22', 22", 22‴, 22ʺʺ).

17. A computer program product comprising computer readable means which, when executed in a computer, will cause the computer to perform the method according to any one of the preceding claims 1 to 8.
